# EUROPEAN PATENT SPECIFICATION

(11) **EP 3 143 954 B1**
(45) Date of publication and mention of the grant of the patent: **14.03.2018**
(21) Application number: 15792739.3
(22) Date of filing: 27.01.2015
(51) Int. Cl.: A61B 17/56, A61B 17/16

(54) **CUTTING HEAD OF EXPANDABLE ORTHOSIS DEVICE**
SCHNEIDEKOPF FÜR EINE EXPANDIERBARE ORTHESEVORRICHTUNG
TÊTE DE COUPE DE DISPOSITIF D'ORTHÈSE EXTENSIBLE

(30) Priority: 14.05.2014 CN 201410201744
(43) Date of publication of application: 22.03.2017
(73) Proprietor: Shandong Guanlong Medical Utensils Co., Ltd., Jinan, Shandong 250101 (CN)
(72) Inventor: YANG, Wenzhou, Jinan Shandong 250101 (CN); SHAO, Weixing, Jinan Shandong 250101 (CN)
(74) Representative: Sun, Yiming
(86) International application number: PCT/CN2015/000047
(87) International publication number: WO 2015/172572

(56) References cited:
- CN-A- 101 984 924
- CN-A- 102 949 777
- CN-A- 104 014 071
- CN-U- 201 855 299
- CN-U- 202 036 672
- CN-U- 202 036 672
- CN-U- 203 018 558
- CN-U- 203 122 569
- CN-U- 203 122 570
- CN-U- 204 017 118
- US-A1- 2010 249 785
- US-A1- 2013 165 935

## Description

### BACKGROUND

### Technical Field

The present invention relates to a surgical instrument for an orthopedics medical surgery, and in particular, to a cutting head of an expansion orthosis.

### Related Art

Currently, for patients with vertebral compression fractures, to relieve pain of the patients, a surgical treatment of vertebroplasty needs to be performed. However, in the surgery of vertebroplasty, a balloon expander needs to be used to expand in a vertebral body to form a cavity for bone cement injection. However, in surgery, because bone spurs in the vertebral body usually pierce the balloon, balloon rupture results in extended surgery time and increased surgery costs. To prevent the balloon from being pierced by the bone spurs, an expansion orthosis is generally used to expand and rotate in the vertebral body to remove the bone spurs in the vertebral body. A utility model entitled "WORKING HEAD OF EXPANSION ORTHOSIS" with application No. 201120086384.5, which was published as CN202036672U, is exactly a technical solution for solving the problem. However, as regards the solution, distances between top ends of cutting knife bulging ports of two cutting knives and a seal end of an outer bushing are the same, and therefore spaces that the two cutting knives can cut in an axial direction are the same, and the two knives are not applicable to cutting a large axial space, which is the drawback of the prior art.

### SUMMARY

An objective of the present invention is to provide a technical solution of a cutting head of an expansion orthosis for the drawback of the prior art. By means of the solution, a difference exists between distances between top ends of two cutting knife bulging ports and a seal end of an outer bushing, so that a cutting space of cutting knives in an axial direction is enlarged.

The present solution is implemented by means of the following technical means: a cutting head of an expansion orthosis, including an outer bushing; a push-pull rod is provided in an inner cavity of the outer bushing; two corresponding metal sheet cutting knives are fixed on a top end of the push-pull rod; a seal head of the outer bushing is provided on a top end of the outer bushing; two strip-shaped cutting knife bulging ports corresponding to the two metal sheet cutting knives are provided on side walls of the outer bushing; the two metal sheet cutting knives can bulge out at the corresponding two cutting knife bulging ports; a central baffle between the two metal sheet cutting knives is further fixed on the seal head of the outer bushing. The present solution is characterized in that distances between top ends of the two cutting knife bulging ports and the seal end of the outer bushing are different. The present solution further has the specific feature that the difference between the distances between the top ends of the two cutting knife bulging ports and the seal end of the outer bushing ranges from 2 mm to 20 mm.

According to the description of the foregoing solution, a beneficial effect of the present solution can be known. Because in the solution, a structure in which a difference exists between distances between top ends of two cutting knife bulging ports and a seal end of an outer bushing is used, maximum radiuses by which two cutting knives bulge when the two cutting knives bulge at the cutting knife bulging port are not on a sample plane, so that an axial cutting space is enlarged. Based on the above, compared with the prior art, the present invention has prominent substantive features and represents a notable program, and the beneficial effect of implementation of the present invention is also obvious.

### BRIEF DESCRIPTION OF THE DRAWINGS

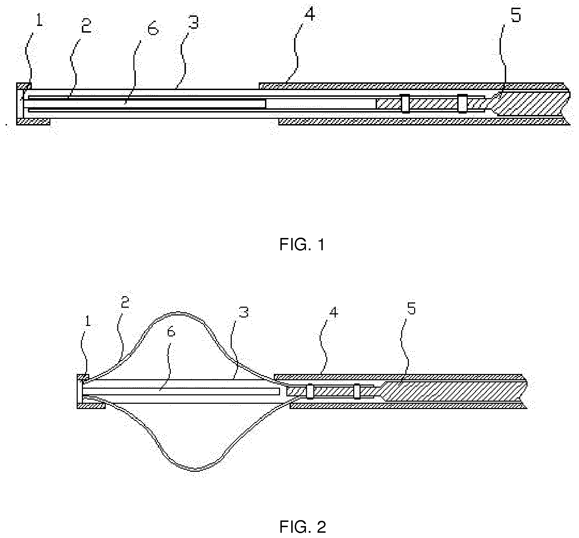

FIG. 1 is a partial sectional schematic structural diagram of a specific implementation manner of the present invention.
FIG. 2 is a partial sectional schematic structural diagram of a working state of FIG. 1.

In the drawings, 1 represents a seal end of an outer bushing; 2 represents a metal sheet cutting knife; 3 represents a cutting knife bulging port; 4 represents an outer bushing; 5 represents a push-pull rod; and 6 represents a central baffle vertebral body.

### DETAILED DESCRIPTION

To clearly describe technical features of the present solution, the present solution is illustrated below by means of a specific implementation manner with reference to the accompanying drawings thereof.

According to the accompanying drawings, it can be seen that in the present solution, there is an outer bushing 4; a push-pull rod 5 is provided in an inner cavity of the outer bushing 4; two corresponding metal sheet cutting knives 2 are fixed on a top end of the push-pull rod 5; a seal head 1 of the outer bushing is provided on a top end of the outer bushing 4; strip-shaped cutting knife bulging ports 3 corresponding to the two metal sheet cutting knives 2 are provided on side walls of the outer bushing 4; the two metal sheet cutting knives 2 can bulge out at the corresponding two cutting knife bulging ports 3; a central baffle 6 between the two metal sheet cutting knives 2 is further fixed on the seal head 1 of the outer bushing; in the present solution, distances between top ends of the two cutting knife bulging ports 3 and the seal end 1 of the outer bushing are different, and the difference ranges from 2 mm to 20 mm, and the difference used in the present specific implementation manner is 10 mm.

## Claims

1. A cutting head of an expansion orthosis for an orthopedics medical surgery, comprising an outer bushing (4); a push-pull rod (5) provided in an inner cavity of the outer bushing; two corresponding metal sheet cutting knives (2) fixed on a top end of the push-pull rod; wherein a seal head (1) of the outer bushing is provided on a top end of the outer bushing; wherein two strip-shaped cutting knife bulging ports (3) corresponding to the two metal sheet cutting knives are provided on side walls of the outer bushing; wherein the two metal sheet cutting knives can bulge out at the corresponding two cutting knife bulging ports; wherein a central baffle (6) between the two metal sheet cutting knives is further fixed on the seal head of the outer bushing, **characterized in that** distances between top ends of the two cutting knife bulging ports and the seal end of the outer bushing are different.

2. The cutting head of an expansion orthosis according to claim 1, **characterized in that**: the difference between the distances between the top ends of the two cutting knife bulging ports and the seal end of the outer bushing ranges from 2 mm to 20 mm.

## Patentansprüche

1. Schneidkopf einer Expansionsorthese für einen orthopädischen medizinischen Eingriff, umfassend eine äußere Hülse (4); eine in einem inneren Hohlraum der äußeren Hülse angeordnete Schub-Zug-Stange (5); zwei entsprechende Metallblechschneidmesser (2), die an einem oberen Ende der Schub-Zug-Stange befestigt sind; wobei ein Dichtkopf (1) der äußeren Hülse an einem oberen Ende der äußeren Hülse angeordnet ist; wobei zwei streifenförmige Schneidmesser-Ausbauchöffnungen (3), die den beiden Metallblechschneidmessern entsprechen, an Seitenwänden der äußeren Hülse angeordnet sind; wobei sich die beiden Metallblechschneidmesser an den entsprechenden beiden Schneidmesser-Ausbauchöffnungen nach außen wölben können; wobei ein mittiges Trennelement (6) zwischen den beiden Metallblechschneidmessern ferner an dem Dichtkopf der äußeren Hülse befestigt ist, **dadurch gekennzeichnet, dass** Abstände zwischen oberen Enden der beiden Schneidmesser-Ausbauchöffnungen und dem Dichtungsende der äußeren Hülse unterschiedlich sind.

2. Schneidkopf einer Expansionsorthese nach Anspruch 1, **dadurch gekennzeichnet, dass**: der Unterschied zwischen den Abständen zwischen den oberen Enden der beiden Schneidmesser-Ausbauchöffnungen und dem Dichtungsende der äußeren Hülse zwischen 2 mm und 20 mm liegt.

## Revendications

1. Tête de coupe d'une orthèse expansible pour chirurgie orthopédique médicale, comprenant une bague extérieure (4); une barre de poussée et traction (5) prévue dans une cavité interne de la bague extérieure ; deux lames de coupe correspondantes en feuillard métallique (2) fixées à une extrémité supérieure de la barre de poussée et traction ; une tête d'étanchéité (1) de la bague extérieure étant prévue à une extrémité supérieure de la bague extérieure ; deux ports de renflement de lame de coupe en forme de bandes (3) correspondant aux lames de coupe en feuillard métallique étant prévus sur des parois latérales de la bague extérieure ; les deux lames de coupe en feuillard métallique pouvant se renfler vers l'extérieur au niveau des deux ports de renflement de lames de coupe correspondants ; un déflecteur central (6) entre les deux lames de coupe en feuillard métallique étant en outre fixé sur la tête d'étanchéité de la bague extérieure, **caractérisée en ce que** les distances entre les extrémités supérieures des deux bords de renflement de lame la bague de coupe et l'extrémité d'étanchéité de la bague extérieure sont différentes.

2. Tête de coupe d'une orthèse expansible selon la revendication 1, **caractérisée en ce que** la distance entre les distances séparant les extrémités supérieures des deux ports de renflement de lame de coupe et la tête d'étanchéité de la bague extérieure va de 2 mm à 20 mm.
